# EUROPEAN PATENT SPECIFICATION

(11) **EP 2 821 090 B1**
(45) Date of publication and mention of the grant of the patent: **09.08.2017**
(21) Application number: 13754406.0
(22) Date of filing: 26.02.2013
(51) Int. Cl.: A61M 5/315, A61M 5/31, A61M 5/50, A61M 5/32

(54) **RETRACTABLE SELF-DESTRUCTING SAFETY SYRINGE WITH REPLACEABLE NEEDLE**
SICHERE, SELBSTZERSTÖRENDE SPRITZE MIT EINZIEHBARER, AUSTAUSCHBARER NADEL
SERINGUE DE SÉCURITÉ RÉTRACTABLE À AUTODESTRUCTION DOTÉE D'AIGUILLE REMPLAÇABLE

(30) Priority: 29.02.2012 CN 201220070623 U
(43) Date of publication of application: 07.01.2015
(73) Proprietor: Wang, Zuyang, Shanghai 200336 (CN)
(72) Inventor: Wang, Zuyang, Shanghai 200336 (CN)
(74) Representative: Petraz, Gilberto Luigi
(86) International application number: PCT/CN2013/000190
(87) International publication number: WO 2013/127259

(56) References cited:
- WO-A1-2005/120608
- WO-A2-2008/136775
- CN-A- 1 803 212
- CN-A- 101 810 900
- CN-A- 102 284 112
- CN-A- 102 327 658
- CN-A- 102 813 981
- CN-A- 102 813 982
- CN-A- 102 813 985
- CN-A- 102 886 089
- CN-U- 202 105 269
- CN-U- 202 236 663
- CN-U- 202 376 562
- CN-U- 202 569 090
- CN-Y- 200 945 295
- US-A- 5 242 400
- US-A- 5 330 440
- US-A1- 2002 177 820
- US-A1- 2003 040 721
- US-A1- 2004 186 427
- US-A1- 2009 171 285

## Description

### BACKGROUND OF THE INVENTION

### FIELD OF THE INVENTION

The present invention relates to a technical field of medical devices, more particularly to a retractable self-destructing safety syringe with replaceable needle, and especially to the mechanical connection structure thereof.

### DESCRPTION OF RELATED ART

Reference Document 1, with Patent No.: ZL200710047077.4, by Application Date: 2007.10.16, with Issued No.: CN101411908, the patentee thereof: Shanghai Dingjiantang Biochemical Technology Co., Ltd, has disclosed a disposable safety vaccine syringe comprising a barrel body, a plunger disposed in the barrel body, a rubber piston disposed near an end of the plunger, a needle disposed in a barrel neck of the barrel body, and a needle disposed on the needle base, which is characterized in that: a lateral notch is formed on a side wall of the end of a plunger body, which is interconnected with a vertical notch formed at the end of the plunger body of the plunger and coupled with the vertical notch; an upper part and a lower part of the needle base are sleeved together to form the needle base, wherein the lower needle base is cylindrical and has a hollow cavity and a lower needle base rib is disposed on an inner wall of the lower needle base and located near an opening of the end of the lower needle base opposite the end at which the upper needle base is disposed; an elastic member formed by interconnecting the lateral notch with the vertical notch is disposed at the end of the plunger where the plunger body has a specific elasticity, wherein the vertical notch is narrow at the top and wide at the bottom while the outer wall of the plunder body above the outer opening of the lateral notch protrudes beyond an outer wall of the plunger body therebelow, the outer opening of the lateral notch is high and the inner opening thereof is low, and the end of the plunger is in a curved shape; the upper needle base is cylindrical, wherein a plurality of reinforcing fins is disposed on the outer wall of the upper portion of the upper needle base and a flange ring of the upper needle base is disposed on the outer wall of the lower portion of the upper needle base, and the outer diameter of the flange ring of the upper needle base is greater than the diameter of the outer wall of the upper needle base by 0.05mm to 0.3mm;the lower needle base is cylindrical and has a hollow cavity, wherein an annular locking groove of the lower needle base is disposed on the inner wall of the cavity of the lower needle base and is coupled with the flange ring of the upper needle base, the inner diameter of the cavity above the annular locking groove of the lower needle base is less than the outer diameter of the outer wall of the plunger body above the flange ring of the upper needle base by 0.1mm to 0.35mm while the inner diameter of the cavity below the annular locking groove of the lower needle base is less than or equals to the outer diameter of the outer wall of the plunger body below the flange ring of the upper needle base, and an annular rib of the lower needle base is disposed on the outer wall of an end of the lower needle base opposite the end of the lower needle base rib; an upper stopping ring of the barrel neck and a lower stopping ring of the barrel neck are respectively disposed on the inner wall of the barrel neck of the barrel body and the distance between the upper stopping ring of the barrel neck and the lower stopping ring of the barrel neck is ranged from 0.3mm to 1.5mm, wherein the upper stopping ring of the barrel neck protrudes beyond the inner wall between the upper stopping ring of the barrel neck and the lower stopping ring of the barrel neck by 0.1mm to 0.6mm, the lower stopping ring of the barrel neck protrudes beyond the inner wall between the upper stopping ring of the barrel neck and the lower stopping ring of the barrel neck by 0.02mm to 0.25mm, the inner diameter of the barrel cavity above the upper stopping ring of the barrel neck is greater than or equals to an inner diameter of the barrel cavity between the upper stopping ring of the barrel neck and the lower stopping ring of the barrel neck while the inner diameter of the barrel cavity between the upper stopping ring of the barrel neck and the lower stopping ring of the barrel neck is less than or equals to the inner diameter of the barrel cavity below the lower stopping ring of the barrel neck, and the inner diameter of the barrel cavity between the upper stopping ring of the barrel neck and the lower stopping ring of the barrel neck is less than the outer diameter of the annular rib of the lower needle base by 0.1mm to 0.5mm; a stopping rib of the barrel body is disposed on the inner wall of the barrel cavity at an end opposite the end of the barrel neck and can be a linear annular rib, an annular rib consisting of several vertical ribs, a vertical rib, or an annular rib consisting of multiple protruding points; and an inner rib of the barrel body is disposed on the inner wall of the barrel body below the stopping rib of the barrel body, wherein the distance between the stopping rib of the barrel body and the inner rib of the barrel body is greater than or equals to the thickness of the annular pre-stopper piece and the inner diameter of the stopping rib of the barrel body is smaller than the diameter of the annular pre-stopper piece.

Reference Document 2, with Patent No.: ZL200720067715.4, by Application Date: 2007.03.08, with Issued No.: CN201076648, the patentee thereof: Shanghai Dingjiantang Biochemical Technology Co., Ltd., has disclosed "a plunger with a metal hook" comprising a plunger body, a columnar locking member disposed on the upper end of the plunger body, and a rubber piston sleeved with the columnar locking member, which is characterized in that a mounting hole with a hook inside is disposed on the end of the plunger body at the position corresponding to the rubber piston. An analogous plunger provided with a metal hook and a safety syringe comprising the same are described in US2003/0040721.

US20040186427 discloses a safety syringe comprising: a cylinder defining a space for accommodating injection liquid and having a neck extending upwardly from an upper end thereof; a plunger inserted inside the cylinder while being movable in the cylinder; an adapter coupled to the neck of the cylinder; and an injection needle connected to the adapter, wherein: the plunger is provided at its upper surface with a vertical rod extending upwardly from an eccentric position with respect with a central axis of the cylinder; the adapter is defined at its interior space with a cavity, into which the rod is inserted while being bent inwardly at its upper end in accordance with the upward movement of the plunger; the adapter is threadedly coupled to the neck of the cylinder; and coupling means are formed at the adapter and plunger, respectively, and adapted to allow the adapter to be rotated and then moved downwardly in accordance with the successive rotating and downward movements of the plunger, thereby causing the injection needle to be withdrawn into the cylinder. In this document, the injection needle is pulled back into the cylinder through the coupling means formed at the adapter and plunger. The barrel is in a vacuum environment after medicine inside the barrel is ejected out of the barrel.

Current safety syringes bear a function of retracting the needle by the hook to realize safe injections. The hook is generally made of metal pieces or plastic pieces. The metal piece requires manufacture process so as to match or be assembled on the plunger. The manufacturing process is complicated and difficult plus uncertainty of stability. Not only are the manufacturing process and cost increased, but the metal piece itself and the assembly process thereof will increase the uncertainty of risks in respect of product safety and quality. The current plastic hook still has structural defects or deficiencies, thereby leading to safety risks or vulnerabilities during use. Though the design of current plastic locking can fulfill the needle-retracting function, the needle still remains on the regular track after being retracted during use, so that it is possible to push out the needle again, which poses a risk of secondary injuries to the user.

In addition, a common problem existing in both current retractable disposable safety syringes with a single needle base and current safety syringes is how to stabilize the locking stability between an inner needle base and a barrel body to the extent satisfying stability requirements, meanwhile for the convenient use purpose, the pulling force for retracting the needle base should not be excessive. Although the rigid locking structures can achieve a stable locking effect between the inner needle base and the inner wall of the barrel body, it is hard or even impossible to meet the requirement for convenient use at the same time. The instability of plastic materials and the tendency to be easily affected by environmental temperature may lead to poor stability of the products with rigid locking structures, which makes it quite difficult to control the quality and precision during production processes and makes it impossible to realize mass production.

As mentioned above, it is still necessary to further improve the structure of the plunger and the locking structure between the inner needle base and the barrel body of the current retractable syringe, thereby further improving safety and stability during use, and achieving the purposes of simplified manufacturing process and reduced manufacturing cost.

### BRIEF SUMMARY OF THE INVENTION

The object of the present invention is to provide retractable self-destructing safety syringe with replaceable needle. An inner needle base inside the syringe and a barrel body of the syringe are interlocked with each other by an elastic dampening engaging mechanism. The locking and releasing effects therebetween are perfectly achieved by skillfully utilizing the dampening gap at the locking position and the plasticity of the locking parts. The elastic dampening engaging mechanism can be formed either on the inner needle base or on the inner wall of the barrel body, meanwhile, an corresponding engagement structure is formed either on the inner wall of the barrel body or on the inner needle base. An end of the plunger body is provided with an asymmetric locking member which has elastic functionality and can be interlocked with the cavity of the needle base of the syringe through engagement. After being retracted back into the barrel body of the syringe, the needle base is forced to deflect toward to one side under a force generated from the asymmetrical elastic mechanism, the needle therefore inclines in a direction nearly a 45 degree angle to one side of the inner wall of the barrel body and is blocked by the barrel neck of the barrel body, so that the needle and the needle base could not be pushed out of the barrel neck again. Therefore, the needle is effectively prevented from being pushed out of the barrel again which may cause secondary injuries, so that to achieve operating safety and self-destruction of the syringe, thereby to overcome and improve defects and deficiencies in the prior arts.

In order to achieve above objects, the main technical solution of the present invention is to provide a retractable self-destructing safety syringe with replaceable needle. The retractable self-destructing safety syringe with replaceable needle comprises a barrel body, a needle base body disposed in the barrel neck at the end of the barrel body, a plunger disposed in the barrel body, a rubber piston disposed near the end of the plunger. It is characterized in that: An elastic dampening engaging mechanism is formed between the barrel neck and the needle base body, which can be disposed either on an outer wall of the needle base body or on an inner wall of the barrel neck.

The present invention discloses a retractable self-destructing safety syringe with replaceable needle according to claim 1. The asymmetric locking member with elastic function is disposed at the end of the plunger. A combination design of the asymmetrical structure with elastic functionality can ensure that the asymmetric locking member can be smoothly inserted into the cavity of the needle base. And the interlocking between the asymmetrical locking member and a locking part of the sleeve-type retractable locking member in the needle base cavity is realized through a elastic expansion mechanism. Since the annular recess in the cavity of the sleeve-type retractable locking member also has elastic and dampening characteristic, when it contacts the asymmetric elastic locking member with being pushed, dampening and deformation correspondingly happens to both, so that the asymmetric elastic locking member can stick into the cavity of the sleeve-type retractable locking member and be interlocked with the sleeve-type retractable locking member when the asymmetric elastic locking member is being retracted. After being retracted back into the barrel body of the syringe, the needle base will be deflected to a side under a asymmetrical force, the needle therefore inclines in a direction nearly a 45 degree angle to one side of the inner wall of the barrel body and is blocked by the barrel neck of the barrel body, so that the needle and the needle base cannot be pushed out of the barrel neck again. Therefore, the needle is prevented from being pushed out of the barrel neck again which may cause secondary injuries to achieve operating safety and self-destruction of the syringe. In the embodiment of the present invention, the asymmetric elastic locking member is a plastic hook. The design of the plastic hook adopts a combination of asymmetrical and elastic structure together with applications of specific angles and sizes, and enables the plastic hook to perform both functions of dampening and interlocking. The above mentioned asymmetric elastic locking member is integrated together with the plunger body and is formed by one-time molding process, which has no need for separated manufacturing and follow-up assembly, and therefore significantly reduced manufacture costs and simplified assembly processes and procedures. The product is easy to use and functionally stable. Interlocking function between the asymmetric elastic locking member at the end of the plunger and the cavity of the inner needle base is very effective and stable. Overall and integral structure design of the product is ingenious with reasonable rationale. The product is easy to use and has wide application potentials. In comparison with designs and techniques of current products, the present invention has significant technical breakthroughs and brought the product unique safety features and practicability.

### BRIEF DESCRIPTION OF THE DRAWINGS

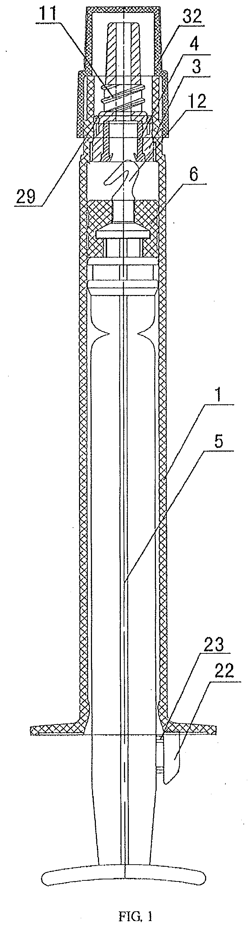

FIG. 1 is a schematic view of the present invention.

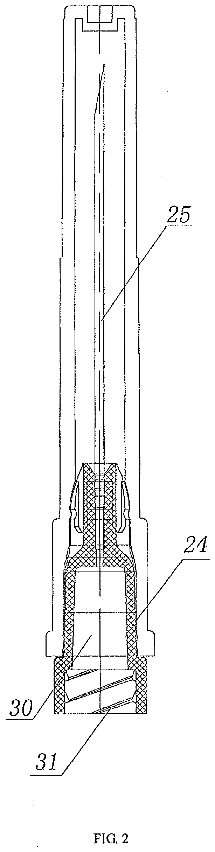

FIG. 2 is schematic diagram of a replaceable of the present invention.

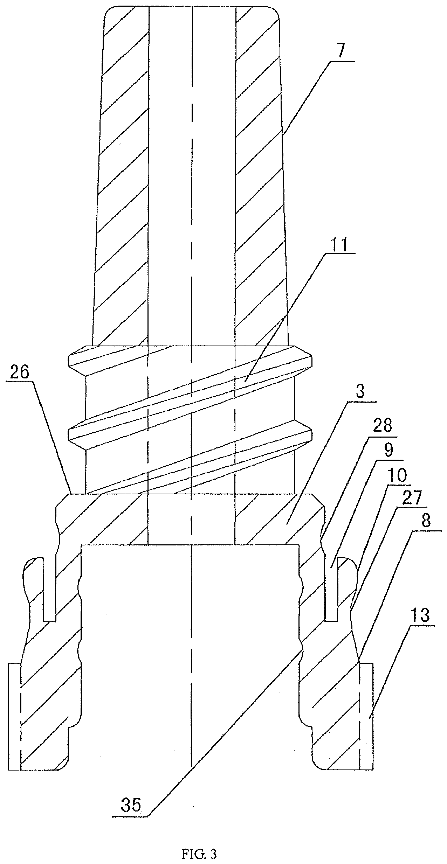

FIG. 3 is a schematic diagram of an needle base body of the present invention.

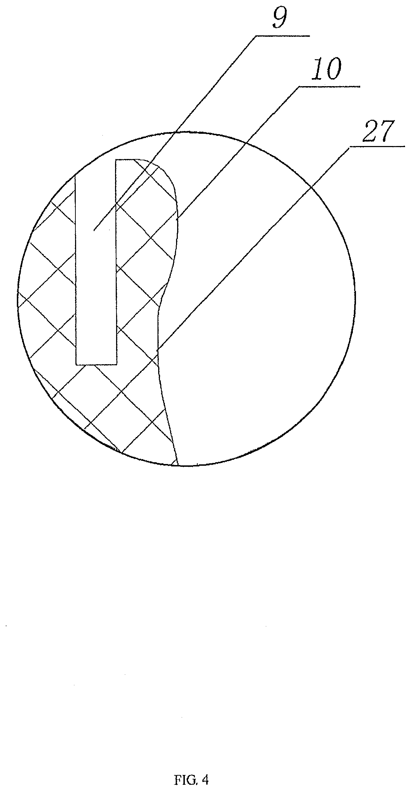

FIG. 4 is an enlarged view of FIG. 3 of the present invention.

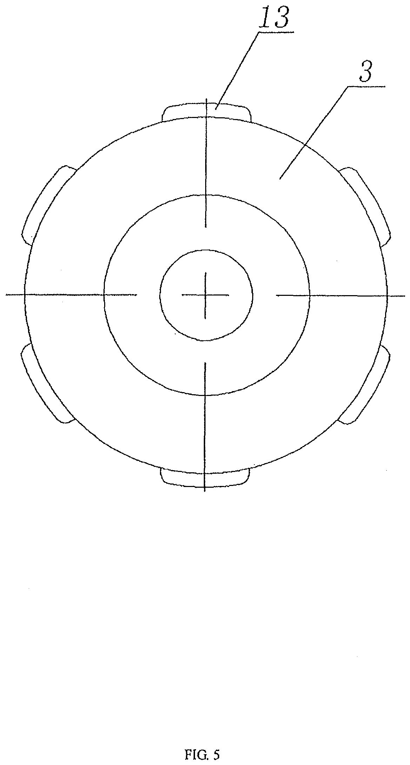

FIG. 5 is a top view of FIG. 3 of the present invention.

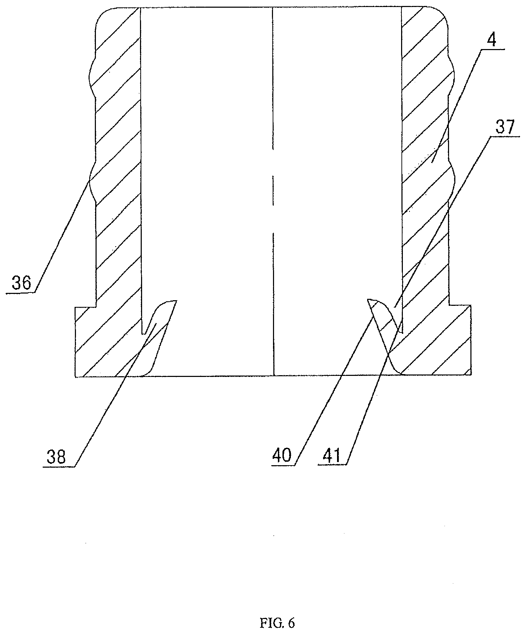

FIG. 6 is a schematic diagram of a internal sleeved retracting locking member of the present invention.

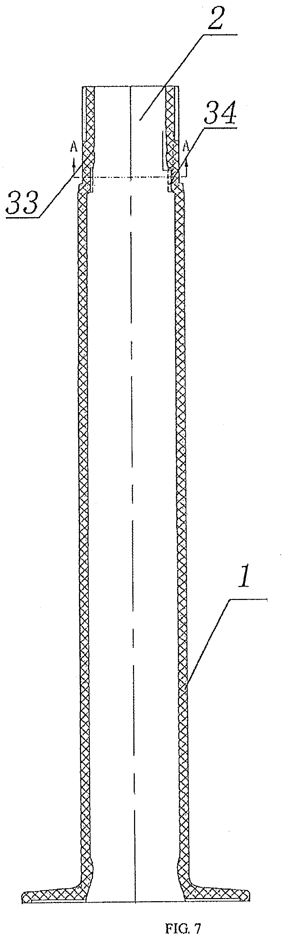

FIG. 7 is a schematic diagram of a barrel body of the present invention.

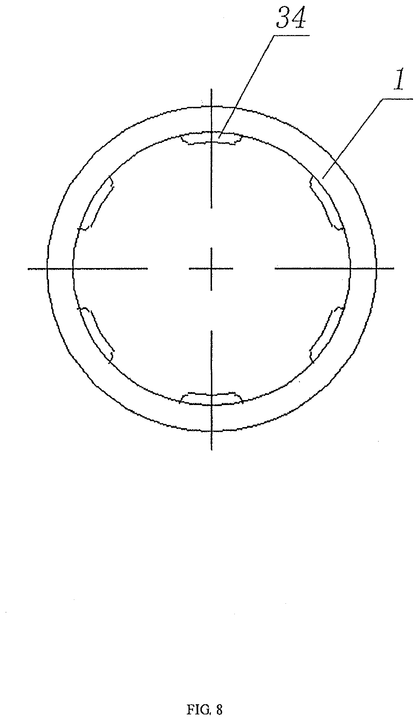

FIG. 8 is a cross-sectional view of FIG. 7 along A-A direction of the present invention.

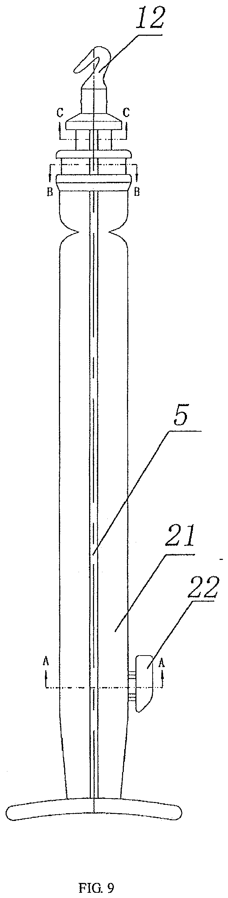

FIG. 9 is a schematic diagram of a plunger of the present invention.

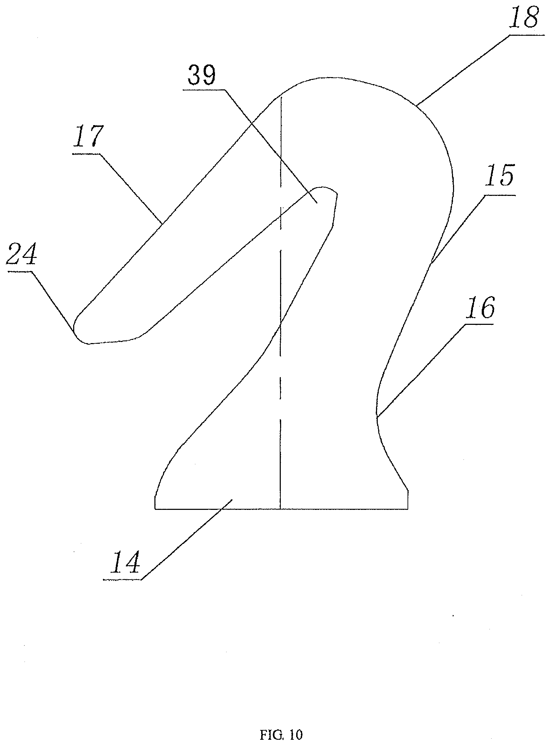

FIG. 10 is a schematic diagram of a hook body of the present invention.

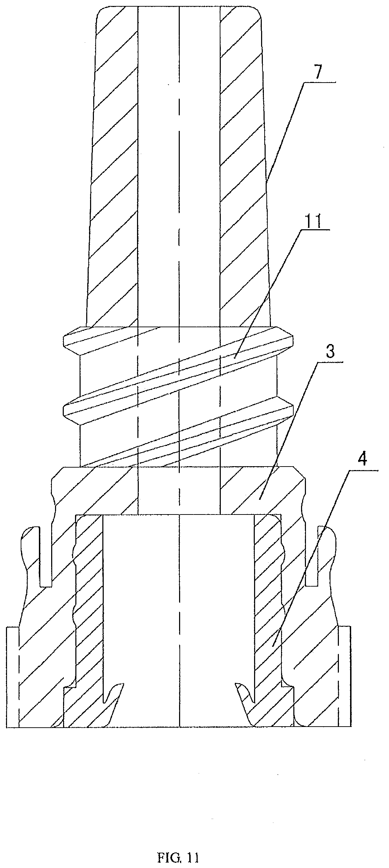

FIG. 11 is an schematic diagram of assembly of the needle base body and the internal sleeved retracting locking member of the present invention.
FIG. 12 is a view of a first use state of the present invention.
FIG. 13 is a view of a second use state of the present invention.
FIG. 14 is a view of a third use state of the present invention.
FIG. 15 is a view of a fourth use state of the present invention.
FIG. 16 is a view of a fifth use state of the present invention.
FIG. 17 is a view of a sixth use state of the present invention.

### DETAILED DESCRIPTION OF THE PREFERRED EMBODIMENT

Hereinafter, preferred embodiments of the present disclosure will be described in detail with reference to the accompanying drawings.

The present invention is a retractable self-destructing safety syringe with replaceable needle, according to claim 1, which comprises a barrel body 1, a needle base body 3 disposed in the barrel neck 2 at the end of the barrel body 1, a plunger 5 disposed in the barrel body 1, and a rubber piston 6 disposed near the end of the plunger 5. It differs from current techniques in that: an elastic dampening engaging mechanism is formed between the barrel neck 2 and the needle base body 3, which can be disposed either on an outer wall of the needle base body 3 or on an inner wall of the barrel neck 2. The needle base body and the barrel body can be interlocked stably and released easily due to the elastic dampening engaging mechanism.

In an embodiment of the present invention, the elastic dampening engaging mechanism is disposed on the needle base body 3. The needle base body 3 has a hollow cavity in where an internal sleeved retracting locking member 4 is disposed, or an exterior of the needle base body 3 is sleeved to a sleeve-type retracting locking member.A replaceable needle assembly 7 is formed on an upper portion of the needle base body 3 and an elastic sealing stopper 8 is formed on a lower portion of the needle base body 3, wherein the replaceable needle assembly 7 and the elastic sealing stopper 8 are integrated together to form a single structure. An annular dampening gap 9 is defined by the surface of the elastic sealing stopper 8, a ring-shaped engagement part 10 is formed on an outer wall of the elastic sealing stopper 8 outside the annular dampening gap 9, anti-rotation ribs 13 are formed on the outer wall of the elastic sealing stopper 8, an external thread assembly 11 is formed on the replaceable needle assembly 7, and an asymmetrical elastic retracting locking member is disposed at the end of the plunger 5.

In another embodiment of the present invention, the elastic dampening engaging mechanism is disposed on the needle base body 3. The needle base body 3 has a hollow cavity in where an internal sleeved retracting locking member 4 is disposed, or an exterior of the needle base body 3 is sleeved to a sleeve-type retracting locking member. A replaceable needle assembly 7 is formed on an upper portion of the needle base body 3 and an elastic sealing stopper 8 is formed on a lower portion of the needle base body 3, wherein the replaceable needle assembly 7 and the elastic sealing stopper 8 are independent parts to form a split assembly structure. An annular dampening gap 9 is defined by a surface of the elastic sealing stopper 8, a ring-shaped engagement part 10 is formed on an outer wall of the elastic sealing stopper 8 outside the annular dampening gap 9, anti-rotation ribs 13 are formed on the outer wall of the elastic sealing stopper 8, an external thread assembly 11 is formed on the replaceable needle assembly 7, and an asymmetrical elastic retracting locking member is disposed on an end of the plunger 5.

In the detailed embodiment, the central axis of the asymmetrical elastic retracting locking member is on a symmetric central line of the plunger, an locking portion is respectively formed on both sides of the central axis and the locking portions on the both sides are asymmetrical to each other, wherein one of the locking portions on one side of the central axis is higher or lower than the other locking portion on the other side, and the width of one of the locking portions on one side of the central axis is larger or smaller than that of the other locking portion on the other side; an elastic mechanism is formed on the locking portion on one side or the locking portions on both sides, and a distance between an outer edge of the locking portion on one side and the symmetric central line of the plunger is ranged from 1mm to 20mm. Through the above structures, the elastic structure of all or part of the asymmetrical elastic retracting locking member can be achieved so that the asymmetrical elastic retracting locking member can enters the cavity of the needle base body smoothly and be locked with each other completely when retracted. An asymmetrical force can be applied on the needle base body though an asymmetrical locking structure so that the needle base body is deviated to one side after entering into the barrel body 1 for realizing the safety operation of the syringe.

In the detailed embodiment, the asymmetrical elastic retracting locking member is a hook 12. The hook 12 is made of plastic material, which is integrated together with the plunger 5 and is formed by one-time molding process. The uniformity of the materials can ensure the stability of the interlocking function of the plunger and the integral structure can reduce assembly processes, so as to improve production efficiency and reduce manufacturing cost.

In the detailed embodiment, a base portion 14 is formed at a lower end of the hook 12, a first arm 15 deflected to one side is formed on an upper part of the base portion 14, a concave curved angle 16 or a corner is defined by an outer sidewall of the junction between the first arm 15 and the base portion 14, and the concave curved angle 16 is ranged from 45 to 179 degrees. A lower end of the first arm 15 is connected with an upper end of the base portion 14, an upper end of the first arm 15 is connected with one end of a second arm 17 and another end of the second arm 17 is suspended. A curved surface 18 is formed on an outer side of the junction between the first arm 15 and the second arm 17, an inner angle 39 ranged from 2 to 90 degrees is defined by an inner side of the junction between the first arm 15 and the second arm 17. The width of an opening formed between the suspended end of the second arm 17 and the first arm 15 is ranged from 0.5mm to 40mm, the length of the first arm 15 is ranged from 0.5mm to 50mm, and the length of the second arm 17 is ranged from 0.5mm to 60mm. A tip 24 is formed on the suspended end of the second arm 17, and the suspended end of the second arm 17 protrudes beyond an outer edge of the base portion 14.

In the detailed embodiment, four reinforcing ribs 21 arranged in a cross shape and at equal angles are formed on the plunger 5, with a pre-stopper piece 22 disposed on an outer edge of the reinforcing ribs 21. The pre-stopper piece 22 is connected with the reinforcing ribs 21 by at least two connection portions 23. After the syringe is assembled, a top end of the pre-stopper piece 22 is just against on the lower end of the barrel body thereby preventing the plunger from moving forwardly and the syringe from being wasted when the syringe is under an external force before use. In use, the pre-stopper piece 22 can be removed from the reinforcing ribs by simply bending along the connection portions.

In the detailed embodiment, a replaceable needle base 24 is connected with the external thread assembly 11 on the replaceable needle assembly 7, a needle tube 25 is disposed on the center of an upper part of the replaceable needle base 24, a columnar cavity 30 interconnected with the needle tube 25 is formed in the replaceable needle base 24. An internal thread assembly 31 interlocked with the external thread assembly 11 is formed on an inner wall of the cavity of the replaceable needle base 24; an upper portion of the columnar cavity 30 is a truncated cone, and the taper of the truncated cone is 6:100. The taper is an international general parameter which can ensure the sealing performance of the assembly.

In the detailed embodiment, anti-rotation ribs 13 are distributed evenly in an annular shape on the outer wall of the elastic sealing stopper 8, and the ends of the anti-rotation ribs 13 are semicircles or cones.

In the detailed embodiment, an upper portion of the replaceable needle assembly 7 is a truncated cone, the taper of the truncated cone is 6:100, and the external thread assembly 11 is formed on an outer wall of a lower portion of the replaceable needle assembly 7. The taper is an international general parameter which can ensure the sealing performance of the assembly.

In the detailed embodiment, an annular step 26 or an annular slope is formed on the transition between the replaceable needle assembly 7 and the elastic sealing stopper 8.

In the detailed embodiment, the annular dampening gap 9 is an integrated annular groove-like structure or a non-integrated annular with segmental structures. The height from an opening of the groove to a bottom thereof is ranged from 0.05mm to 4mm, the width of the annular dampening gap 9 is ranged from 0.05mm to 4mm, the distance from an outer groove wall of the annular dampening gap 9 to a top of a curved surface of the ring-shaped engagement part 10 is ranged from 0.1mm to 6mm, the distance from the opening of the outer groove wall of the annular dampening gap 9 to the outer wall of the ring-shaped engagement part 10 is ranged from 0.1mm to 6mm, and the distance from the bottom of the outer groove wall of the annular dampening gap 9 to the outer wall of the ring-shaped engagement part 10 is ranged from 0.1mm to 6mm. The surface of the ring-shaped engagement part 10 is a curved surface, and the distance from the top of the curved surface of the ring-shaped engagement part 10 to an upper edge of the curved surface is less than the distance from the top of the curved surface of the ring-shaped engagement part 10 to a lower edge of the curved surface. An annular recess 27 is formed on the outer wall of the elastic sealing stopper 8 below the ring-shaped engagement part 10.

In the detailed embodiment, the annular recess 27 is in the same horizontal plane as a groove bottom of the annular dampening gap 9, or higher than a groove bottom of the annular dampening gap 9.

In the detailed embodiment, an O-ring assembly groove 28 is formed on an outer wall of the elastic sealing stopper 8 below the annular step 26, and an O-ring sealing gasket 29 is assembled into the O-ring assembly groove 28.

In the detailed embodiment, the exterior of the barrel neck 2 of the barrel body 1 is covered by a barrel body cap 32 for protecting the end of the barrel body and the end of the needle base body 3.

In the detailed embodiment, an annular engagement sealing recess 33 matching the ring-shaped engagement part 10 is disposed on an inner wall of the barrel neck 2 of the barrel body 1. The depth of the annular engagement sealing recess 33 is ranged from 0.01mm to 4mm and the height of the annular engagement sealing recess 33 is ranged from 0.01mm to 6mm. The recess bottom of the annular engagement sealing recess 33 is a curved surface, and anti-rotation ribs 34 matching the anti-rotation ribs 13 are formed on the inner wall of the barrel neck 2 below the annular engagement sealing recess 33.

In the detailed embodiment, at least one annular stopping groove of the needle base 35 is formed on the inner wall of the cavity of the needle base body 3, the internal sleeved retracting locking member 4 is disposed in the cavity of the needle base body 3, and at least one stopping flange ring 36 matching the annular stopping groove of the needle base 35 is formed on an outer wall of the internal sleeved retracting locking member 4.

In the detailed embodiment, the internal sleeved retracting locking member 4 has a hollow inner cavity, a ring-shaped engaging and locking portion 38 is formed on the inner wall of the cavity near an opening of a lower end of internal sleeved retracting locking member 4. The ring-shaped engaging and locking portion has a certain damping elastic function. When the asymmetrical elastic retracting locking member contacts the ring-shaped engaging and locking portion during the pushing process, elastic dampening deformation occurs to both of them under interactive forces, so that the asymmetrical elastic retracting locking member can smoothly go through the ring-shaped engaging and locking portion in the pushing process.

In the detailed embodiment, an annular recess 37 is formed on an upper part of the ring-shaped engaging and locking portion 38. The thickness of an inner sidewall 40 of the annular recess 37 is ranged from 0.01mm to 3mm, an angle ranged from 2 to 90 degrees is formed between the inner side wall of the annular recess 37 and an outer sidewall 41 of the annular recess 37, and the inner diameter of an upper end of the inner side wall of the annular recess 37 is ranged from 1mm to 80mm. The above mentioned technical parameters ensure the elastic dampening function of the ring-shaped engaging and locking portion 38, which is realized especially at the outer wall 41. In the detailed embodiment, the width of an opening of the annular recess 37 is greater than the width of a bottom thereof.

After the asymmetrical elastic retracting locking member slides into the internal sleeved retracting locking member 4, one side end of the asymmetrical elastic retracting locking member (the suspended end of the second arm) is just blocked into the annular groove 37 when retracting the plunger, so that the end of the plunger is completely engaged with the internal sleeved retracting locking member 4.

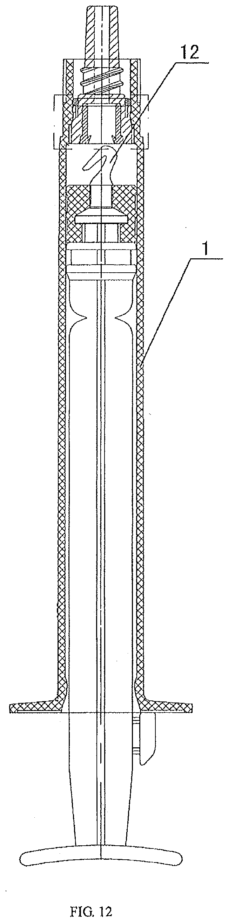

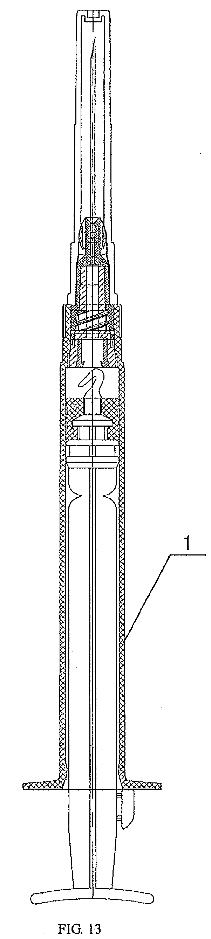

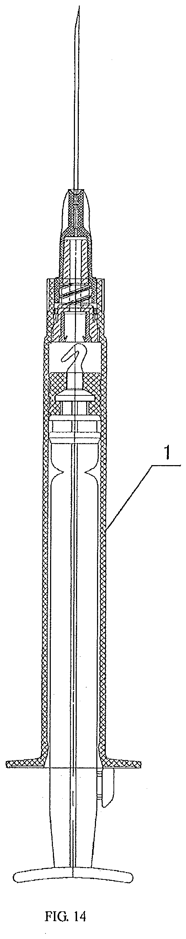

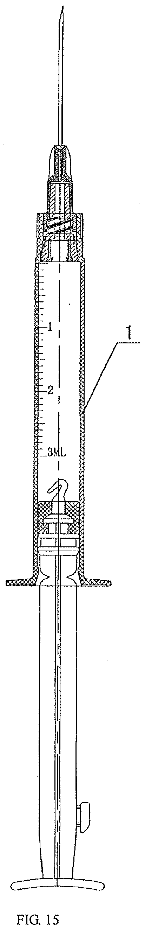

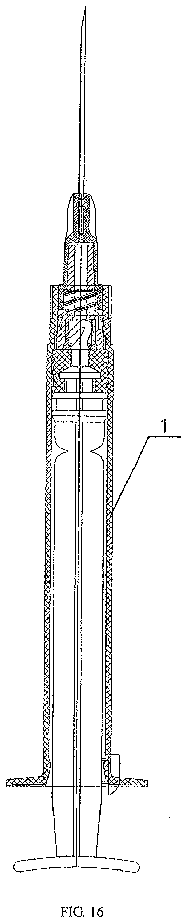

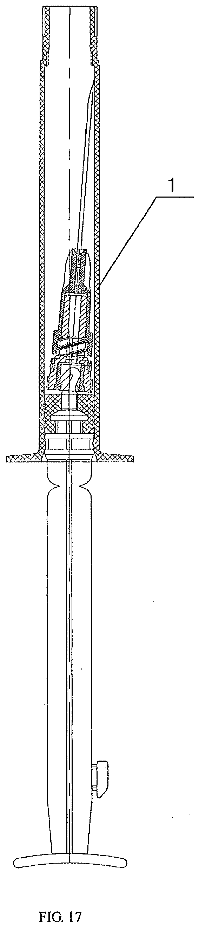

In the practical operation, as shown in FIG. 12 to FIG. 17, the replaceable needle base is rotated into the end of the needle base body. It is realized that both of them are assembled tightly because of the cooperation between the tread structure and the structure with taper 6:100. When the injection begins after drawing the liquid, as slowly pushing the plunger and then contact occurs between the asymmetrical elastic retracting locking member and the internal sleeved retracting locking member, one side of the asymmetrical elastic retracting locking member is deformed so that the width is narrowed and then the asymmetrical elastic retracting locking member enters the cavity of the internal sleeved retracting locking member due to the greater total width of the asymmetrical elastic retracting locking member than the inner diameter of the annular groove 37 in the internal sleeved retracting locking member. After the asymmetrical elastic retracting locking member enters the cavity, the force is removed and the asymmetrical elastic retracting locking member restores to its original state. Pull the plunger backwards when the injection is finished, and the side end of the asymmetrical elastic retracting locking member (the suspended end of the second arm of the hook) is just stuck in the annular groove of the internal sleeved retracting locking member. The annular dampening gap of the elastic sealing stopper on the needle base body is deformed under the pulling force of the plunger that is transferred to the needle base body through the internal sleeved retracting locking member. The ring-shaped engagement part slides out from the annular engagement sealing recess of the barrel body as the annular dampening gap is being narrowed down. Finally, the whole needle is pulled into the cavity of the barrel body. The needle is deflected to one side and deviated from the regular displacement track under the force generated from the asymmetrical elastic retracting locking member, so that the needle cannot be re-assembled through the barrel neck, thereby realizing safe operation and self-destruction of the syringe.

The above content describes the present invention in combination with the detailed preferred embodiment method. However, it should not be considered that the detailed embodiment of the present invention is only limited to the above description. A person of ordinary skill in the art, without departing from the concept of the present invention, may make many simple derivations or alternations, which should be deemed within the scope of the present invention.

## Claims

1. A retractable self-destructing safety syringe with replaceable needle, comprising a barrel body (1), a needle base body (3) disposed in a barrel neck (2) at an end of the barrel body (1), a plunger (5) disposed in the barrel body (1), and a rubber piston (6) disposed near an end of the plunger (5), **characterized in that**: an elastic dampening engaging mechanism is formed between the barrel neck (2) and the needle base body (3), wherein the elastic dampening engaging mechanism is disposed on the needle base body (3) having a hollow cavity in where an internal sleeved retracting locking member (4) is disposed, or an exterior of the needle base body (3) is sleeved to a sleeve-type retracting locking member; a replaceable needle assembly (7) is formed on an upper portion of the needle base body (3) and an elastic sealing stopper (8) is formed on a lower portion of the needle base body (3), wherein the replaceable needle assembly (7) and the elastic sealing stopper (8) are integrated together to form a single structure; an annular dampening gap (9) is defined by a surface of the elastic sealing stopper (8); a ring-shaped engagement part (10) is formed on an outer wall of the elastic sealing stopper (8) outside the annular dampening gap (9); anti-rotation ribs (13) are formed on the outer wall of the elastic sealing stopper (8); an external thread assembly (11) is formed on the replaceable needle assembly (7) and an asymmetrical elastic retracting locking member is disposed at an end of the plunger (5);
wherein the asymmetrical elastic retracting locking member is a hook (12) made of plastic material, and the hook is integrated together with the plunger (5) and is formed by one-time molding process; and
wherein a base portion (14) is formed at a lower end of the hook (12); a first arm (15) deflected to one side is formed on an upper part of the base portion (14); a concave curved angle (16) or a corner is defined by an outer sidewall of a junction between the first arm (15) and the base portion (14), and the concave curved angle (16) is ranged from 45 to 179 degrees; a lower end of the first arm (15) is connected with an upper end of the base portion (14), an upper end of the first arm (15) is connected with one end of a second arm (17) and another end of the second arm (17) is suspended; a curved surface (18) is formed on an outer side of a junction between the first arm (15) and the second arm (17), an inner angle (39) ranged from 2 to 90 degrees is defined by an inner side of the junction between the first arm (15) and the second arm (17); a width of an opening formed between the suspended end of the second arm (17) and the first arm (15) is ranged from 0.5mm to 40mm; the length of the first arm (15) is ranged from 0.5mm to 50mm, and the length of the second arm (17) is ranged from 0.5mm to 60mm.

2. The retractable self-destructing safety syringe with replaceable needle as claimed in claim 1, **characterized in that**: the elastic dampening engaging mechanism is disposed on the needle base body (3) having a hollow cavity in where an internal sleeved retracting locking member (4) is disposed, or an exterior of the needle base body (3) is sleeved to a sleeve-type retracting locking member; a replaceable needle assembly (7) is formed on an upper portion of the needle base body (3) and an elastic sealing stopper (8) is formed on a lower portion of the needle base body (3), wherein the replaceable needle assembly (7) and the elastic sealing stopper (8) are independent parts to form a split assembly structure; an annular dampening gap (9) is defined by a surface of the elastic sealing stopper (8); a ring-shaped engagement part (10) is formed on an outer wall of the elastic sealing stopper (8) outside the annular dampening gap (9); anti-rotation ribs (13) are formed on the outer wall of the elastic sealing stopper (8); an external thread assembly (11) is formed on the replaceable needle assembly (7).

3. The retractable self-destructing safety syringe with replaceable needle as claimed in claim 1, **characterized in that**: a central axis of the asymmetrical elastic retracting locking member is on a symmetric central line of the plunger, an locking portion is respectively formed on both sides of the central axis and the locking portions on the both sides are asymmetrical to each other, wherein one of the locking portions on one side of the central axis is higher or lower than the other locking portion on the other side and the width of one of the locking portions on one side of the central axis is larger or smaller than the width of the other locking portion on the other side; an elastic mechanism is formed on the locking portion on one side or the locking portions on both sides, and a distance between an outer edge of the locking portion on one side and the symmetric central line of the plunger is ranged from 1mm to 20mm.

4. The retractable self-destructing safety syringe with replaceable needle as claimed in claim 1, **characterized in that**: four reinforcing ribs (21) arranged in a cross shape and at equal angles are formed on the plunger (5), with a pre-stopper piece (22) disposed on an outer edge of the reinforcing ribs (21).

5. The retractable self-destructing safety syringe with replaceable needle as claimed in claim 4, **characterized in that**: the pre-stopper piece (22) is connected with the reinforcing ribs (21) by at least two connection portions (23).

6. The retractable self-destructing safety syringe with replaceable needle as claimed in claim 1, **characterized in that**: a tip (24) is formed on the suspended end of the second arm (17), and the suspended end of the second arm (17) protrudes beyond an outer edge of the base portion (14).

7. The retractable self-destructing safety syringe with replaceable needle as claimed in claim 1, **characterized in that**: a replaceable needle base(24) is connected with the external thread assembly (11) on the replaceable needle assembly (7), a needle tube (25) is disposed on a center of an upper part of the replaceable needle base (24); a columnar cavity (30) interconnected with the needle tube (25) is formed in the replaceable needle base (24), an internal thread assembly (31) interlocked with the external thread assembly (11) is formed on an inner wall of the cavity of the replaceable needle base (24), an upper portion of the columnar cavity (30) is a truncated cone, and the taper of the truncated cone is 6:100.

8. The retractable self-destructing safety syringe with replaceable needle as claimed in claim 1, **characterized in that**: anti-rotation ribs (13) are distributed evenly in an annular shape on the outer wall of the elastic sealing stopper (8), and the ends of the anti-rotation ribs (13) are semicircles or cones.

9. The retractable self-destructing safety syringe with replaceable needle as claimed in claim 1, **characterized in that**: an upper portion of the replaceable needle assembly (7) is a truncated cone, the taper of the truncated cone is 6:100, and the external thread assembly (11) is formed on an outer wall of a lower portion of the replaceable needle assembly (7).

10. The retractable self-destructing safety syringe with replaceable needle as claimed in claim 1, **characterized in that**: an annular step (26) or an annular slope is formed on a transition between the replaceable needle assembly (7) and the elastic sealing stopper (8).

11. The retractable self-destructing safety syringe with replaceable needle as claim in claim 1 **characterized in that**: the annular dampening gap (9) is an annular groove-like structure, a height from an opening of the groove to a bottom thereof is ranged from 0.05mm to 4mm, a width of the annular dampening gap (9) is ranged from 0.05mm to 4mm, the distance from an outer groove wall of the annular dampening gap (9) to a top of a curved surface of the ring-shaped engagement part (10) is ranged from 0.1mm to 6mm, the distance from the opening of the outer groove wall of the annular dampening gap (9) to the outer wall of the ring-shaped engagement part (10) is ranged from 0.1mm to 6mm, the distance from the bottom of the outer groove wall of the annular dampening gap (9) to the outer wall of the ring-shaped engagement part (10) is ranged from 0.1mm to 6mm; a surface of the ring-shaped engagement part (10) is a curved surface, and the distance from the top of the curved surface of the ring-shaped engagement part (10) to an upper edge of the curved surface is less than the distance from the top of the curved surface of the ring-shaped engagement part (10) to a lower edge of the curved surface, and an annular recess (27) is formed on the outer wall of the elastic sealing stopper (8) below the ring-shaped engagement part (10).

12. The retractable self-destructing safety syringe with replaceable needle as claimed in claim 11, **characterized in that**: the annular recess (27) is in the same horizontal plane as a groove bottom of the annular dampening gap (9), or higher than a groove bottom of the annular dampening gap (9).

13. The retractable self-destructing safety syringe with replaceable needle according to claim 10, **characterized in that**: an O-ring assembly groove (28) is formed on an outer wall of the elastic sealing stopper (8) below the annular step (26), and an O-ring sealing gasket (29) is assembled into the O-ring assembly groove (28).

14. The retractable self-destructing safety syringe with replaceable needle according to claim 1, **characterized in that**: the exterior of the barrel neck (2) of the barrel body (1) is covered by a barrel body cap (32).

15. The retractable self-destructing safety syringe with replaceable needle as claimed in claim 1, **characterized in that**: an annular engagement sealing recess(33) matching the ring-shaped engagement part (10) is disposed on an inner wall of the barrel neck (2) of the barrel body (1), the depth of the annular engagement sealing recess (33) is ranged from 0.01mm to 4mm and the height of the annular engagement sealing recess (33) is ranged from 0.01mm to 6mm, and a recess bottom of the annular engagement sealing recess (33) is a curved surface, and anti-rotation ribs (34) matching the anti-rotation ribs (13) are formed on the inner wall of the barrel neck (2) below the annular engagement sealing recess (33).

16. The retractable self-destructing safety syringe with replaceable needle as claimed in claim 1, **characterized in that**: at least one annular stopping groove of the needle base (35) is formed on the inner wall of the cavity of the needle base body (3), the internal sleeved retracting locking member (4) is disposed in the cavity of the needle base body (3), and at least one stopping flange ring (36) matching the annular stopping groove inside the needle base (35) is formed on an outer wall of the internal sleeved retracting locking member (4).

17. The retractable self-destructing safety syringe with replaceable needle as claimed in claim 1, **characterized in that**: the internal sleeved retracting locking member (4) has a hollow inner cavity, a ring-shaped engaging and locking portion (38) is formed on the inner wall of the cavity near an opening of a lower end of the internal sleeved retracting locking member (4).

18. The retractable self-destructing safety syringe with replaceable needle as claimed in claim 17, **characterized in that**: an annular recess (37) is formed on an upper part of the ring-shaped engaging and locking portion (38), a thickness of an inner sidewall (40) of the annular recess(37) is ranged from 0.01mm to 3mm, an angle ranged from 2 to 90 degrees is formed between the inner side wall of the annular recess (37) and an outer sidewall (41) of the annular recess (37), and an inner diameter of an upper end of the inner side wall of the annular recess (37) is ranged from 1mm to 80mm.

19. The retractable self-destructing safety syringe with replaceable needle as claimed in claim 18, **characterized in that**: the width of an opening of the annular recess (37) is greater than the width of a bottom thereof.

## Patentansprüche

1. Einziehbare selbstzerstörende Sicherheitsspritze mit austauschbarer Nadel, umfassend einen Zylinderkörper (1), einen Nadelbasiskörper (3), der in einem Zylinderhals (2) an einem Ende des Zylinderkörpers (1) angeordnet ist, einen Stößel (5), der im Zylinderkörper (1) angeordnet ist, und einen Gummikolben (6), der nahe bei einem Ende des Stößels (5) angeordnet ist, **dadurch gekennzeichnet, dass**: ein elastischer Dämpfungs-Eingriffsmechanismus zwischen dem Zylinderhals (2) und dem Nadelbasiskörper (3) geformt ist, worin der elastische Dämpfungs-Eingriffsmechanismus am Nadelbasiskörper (3) angeordnet ist, der einen leeren Hohlraum aufweist, in dem ein innen eingehülstes zurückziehbares Verriegelungselement (4) angeordnet ist, oder eine Außenseite des Nadelbasiskörpers (3) an einem hülsenartigen zurückziehbaren Verriegelungselement umhüllt ist; eine austauschbare Nadelanordnung (7) an einem oberen Teil des Nadelbasiskörpers (3) geformt ist und ein elastischer Abdichtungsstopfen (8) an einem unteren Teil des Nadelbasiskörpers (3) geformt ist, worin die austauschbare Nadelanordnung (7) und der elastische Abdichtungsstopfen (8) zusammen integriert sind, um eine einzige Struktur zu bilden; eine ringartige Dämpfungslücke (9) durch eine Fläche des elastischen Abdichtungsstopfens (8) definiert wird; ein ringförmiges Eingriffsteil (10) an einer Außenwand des elastischen Abdichtungsstopfens (8) außerhalb der ringartigen Dämpfungslücke (9) geformt ist; drehungsverhindernde Rippen (13) an der Außenwand des elastischen Abdichtungsstopfens (8) geformt sind; eine Außengewindeanordnung (11) an der austauschbaren Nadelanordnung (7) geformt ist und ein asymmetrisches elastisches zurückziehbares Verriegelungselement an einem Ende des Stößels (5) angeordnet ist;
worin das asymmetrische elastische zurückziehbare Verriegelungselement ein Haken (12) aus Kunststoff ist, und der Haken zusammen mit dem Stößel (5) integriert ist und durch ein einmaliges Formverfahren geformt ist; und
worin ein Basisabschnitt (14) an einem unteren Ende des Hakens (12) geformt ist; ein erster, zu einer Seite hin abgelenkter Arm (15) an einem oberen Teil des Basisabschnitts (14) geformt ist; ein konkaver gekrümmter Winkel (16) oder eine Ecke durch eine äußere Seitenwand eines Anschlusses zwischen dem ersten Arm (15) und dem Basisabschnitt (14) definiert wird, und der konkave gekrümmte Winkel (16) in einem Bereich zwischen 45 und 179 Grad liegt; ein unteres Ende des ersten Arms (15) mit einem oberen Ende des Basisabschnitts (14) verbunden ist, ein oberes Ende des ersten Arms (15) mit einem Ende eines zweiten Arms (17) verbunden ist und ein anderes Ende des zweiten Arms (17) abgehängt ist; eine gekrümmte Fläche (18) an einer Außenseite eines Anschlusses zwischen dem ersten Arm (15) und dem zweiten Arm (17) geformt ist, ein Innenwinkel (39) in einem Bereich zwischen 2 und 90 Grad durch eine Innenseite des Anschlusses zwischen dem ersten Arm (15) und dem zweiten Arm (17) definiert wird; eine Breite einer Öffnung, die zwischen dem abgehängten Ende des zweiten Arms (17) und dem ersten Arm (15) geformt ist, in einem Bereich zwischen 0,5 mm und 40 mm liegt; die Länge des ersten Arms (15) zwischen 0,5 mm und 50 mm liegt und die Länge des zweiten Arms (17) zwischen 0,5 mm und 60 mm liegt.

2. Einziehbare selbstzerstörende Sicherheitsspritze mit austauschbarer Nadel nach Anspruch 1, **dadurch gekennzeichnet, dass**: der elastische Dämpfungs-Eingriffsmechanismus am Nadelbasiskörper (3) angeordnet ist, der einen leeren Hohlraum aufweist, in dem ein innen eingehülstes zurückziehbares Verriegelungselement (4) angeordnet ist, oder eine Außenseite des Nadelbasiskörpers (3) an einem hülsenartigen zurückziehbaren Verriegelungselement umhüllt ist; eine austauschbare Nadelanordnung (7) an einem oberen Teil des Nadelbasiskörpers (3) geformt ist und ein elastischer Abdichtungsstopfen (8) an einem unteren Teil des Nadelbasiskörpers (3) geformt ist, worin die austauschbare Nadelanordnung (7) und der elastische Abdichtungsstopfen (8) unabhängige Teile sind, um eine geteilte Anordnungsstruktur zu bilden; eine ringartige Dämpfungslücke (9) durch eine Fläche des elastischen Abdichtungsstopfens (8) definiert wird; ein ringförmiges Eingriffsteil (10) an einer Außenwand des elastischen Abdichtungsstopfens (8) außerhalb der ringartigen Dämpfungslücke (9) geformt ist; drehungsverhindernde Rippen (13) an der Außenwand des elastischen Abdichtungsstopfens (8) geformt sind; eine Außengewindeanordnung (11) an der austauschbaren Nadelanordnung (7) geformt ist.

3. Einziehbare selbstzerstörende Sicherheitsspritze mit austauschbarer Nadel nach Anspruch 1, **dadurch gekennzeichnet, dass**: eine Mittelachse des asymmetrischen elastischen zurückziehbaren Verriegelungselements auf einer symmetrischen Mittellinie des Stößels liegt, ein Verriegelungsabschnitt jeweils beiderseits der Mittelachse geformt ist und die Verriegelungsabschnitte auf beiden Seiten asymmetrisch zueinander sind, worin einer der Verriegelungsabschnitte auf einer Seite der Mittelachse höher oder niedriger als der andere Verriegelungsabschnitt auf der anderen Seite ist und die Breite eines der Verriegelungsabschnitte auf einer Seite der Mittelachse größer oder kleiner als die Breite des anderen Verriegelungsabschnitts auf der anderen Seite ist; ein elastischer Mechanismus am Verriegelungsabschnitt auf einer Seite oder an den Verriegelungsabschnitten auf beiden Seiten geformt ist, und ein Abstand zwischen einer Außenkante des Verriegelungsabschnitts auf einer Seite und der symmetrischen Mittellinie des Stößels im Bereich zwischen 1 mm und 20 mm liegt.

4. Einziehbare selbstzerstörende Sicherheitsspritze mit austauschbarer Nadel nach Anspruch 1, **dadurch gekennzeichnet, dass**: vier Verstärkungsrippen (21), die kreuzförmig und in gleichen Winkeln angeordnet sind, am Stößel geformt sind, wobei ein Vorstopfenstück (22) an einer Außenkante der Verstärkungsrippen (21) angeordnet ist.

5. Einziehbare selbstzerstörende Sicherheitsspritze mit austauschbarer Nadel nach Anspruch 4, **dadurch gekennzeichnet, dass**: das Vorstopfenstück (22) mit den Verstärkungsrippen (21) durch zumindest zwei Verbindungsabschnitte (23) verbunden ist.

6. Einziehbare selbstzerstörende Sicherheitsspritze mit austauschbarer Nadel nach Anspruch 1, **dadurch gekennzeichnet, dass**: eine Spitze (24) an dem abgehängten Ende des zweiten Arms (17) geformt ist und das abgehängte Ende des zweiten Arms (17) über eine Außenkante des Basisabschnitts (14) hinausragt.

7. Einziehbare selbstzerstörende Sicherheitsspritze mit austauschbarer Nadel nach Anspruch 1, **dadurch gekennzeichnet, dass**: eine austauschbare Nadelbasis (24) mit der Außengewindeanordnung (11) an der austauschbaren Nadelanordnung (7) verbunden ist, ein Nadelrohr (25) an einem Zentrum eines oberen Teils der austauschbaren Nadelbasis (24) angeordnet ist; ein säulenartiger Hohlraum (30), der mit dem Nadelrohr (25) verbunden ist, in der austauschbaren Nadelbasis (24) geformt ist, eine Innengewindeanordnung (31), die in die Außengewindeanordnung (11) greift, an einer Innenwand des Hohlraums der austauschbaren Nadelbasis (24) geformt ist, ein oberer Abschnitt des säulenartigen Hohlraums (30) ein Kegelstumpf ist und die Verjüngung des Kegelstumpfes 6:100 beträgt.

8. Einziehbare selbstzerstörende Sicherheitsspritze mit austauschbarer Nadel nach Anspruch 1, **dadurch gekennzeichnet, dass**: drehungsverhindernde Rippen (13) gleichmäßig nach einer ringförmigen Form an der Außenwand des elastischen Abdichtungsstopfens (8) verteilt sind, und die Enden der drehungsverhindernden Rippen (13) Halbkreise oder Kegel sind.

9. Einziehbare selbstzerstörende Sicherheitsspritze mit austauschbarer Nadel nach Anspruch 1, **dadurch gekennzeichnet, dass**: ein oberer Abschnitt der austauschbaren Nadelanordnung (7) ein Kegelstumpf ist, die Verjüngung des Kegelstumpfes 6:100 beträgt, und die Außengewindeanordnung (11) an einer Außenwand eines unteren Abschnitts der austauschbaren Nadelanordnung (7) geformt ist.

10. Einziehbare selbstzerstörende Sicherheitsspritze mit austauschbarer Nadel nach Anspruch 1, **dadurch gekennzeichnet, dass**: eine ringförmige Stufe (26) oder eine ringförmige Neigung an einem Übergang zwischen der austauschbaren Nadelanordnung (7) und dem elastischen Abdichtungsstopfen (8) geformt ist.

11. Einziehbare selbstzerstörende Sicherheitsspritze mit austauschbarer Nadel nach Anspruch 1, **dadurch gekennzeichnet, dass**: die ringartige Dämpfungslücke (9) eine ringartige, nutenartige Struktur ist, wobei eine Höhe von einer Öffnung der Nut zu einem Boden derselben im Bereich von 0,05 mm bis 4 mm liegt, eine Breite der ringartigen Dämpfungslücke (9) im Bereich von 0,05 mm bis 4 mm liegt, der Abstand von einer äußeren Nutenwand der ringartigen Dämpfungslücke (9) zu einem Oberteil einer gekrümmten Fläche des ringförmigen Eingriffsteils (10) im Bereich von 0,1 mm bis 6 mm liegt, der Abstand von der Öffnung der äußeren Nutenwand der ringartigen Dämpfungslücke (9) zur Außenwand des ringförmigen Eingriffsteils (10) im Bereich von 0,1 mm bis 6 mm liegt, der Abstand von dem Boden der äußeren Nutenwand der ringartigen Dämpfungslücke (9) zur Außenwand des ringförmigen Eingriffsteils (10) im Bereich von 0,1 mm bis 6 mm liegt; eine Fläche des ringförmigen Eingriffsteils (10) eine gekrümmte Fläche ist, und der Abstand von dem Oberteil der gekrümmten Fläche des ringförmigen Eingriffsteils (10) zu einer Oberkante der gekrümmten Fläche kleiner als der Abstand von dem Oberteil der gekrümmten Fläche des ringförmigen Eingriffsteils (10) zu einer Unterkante der gekrümmten Fläche ist, und eine ringförmige Ausnehmung (27) an der Außenwand des elastischen Abdichtungsstopfens (8) unter dem ringförmigen Eingriffsteil (10) geformt ist.

12. Einziehbare selbstzerstörende Sicherheitsspritze mit austauschbarer Nadel nach Anspruch 11, **dadurch gekennzeichnet, dass**: die ringförmige Ausnehmung (27) in derselben horizontalen Ebene wie ein Nutenboden der ringartigen Dämpfungslücke (9) oder höher als ein Nutenboden der ringartigen Dämpfungslücke (9) liegt.

13. Einziehbare selbstzerstörende Sicherheitsspritze mit austauschbarer Nadel nach Anspruch 10, **dadurch gekennzeichnet, dass**: eine O-Ringmontagenut (28) an einer Außenwand des elastischen Abdichtungsstopfens (8) unter der ringförmigen Stufe (26) geformt ist und eine O-Ringdichtungsmanschette (29) in die O-Ringmontagenut (28) eingebaut ist.

14. Einziehbare selbstzerstörende Sicherheitsspritze mit austauschbarer Nadel nach Anspruch 1, **dadurch gekennzeichnet, dass**: die Außenseite des Zylinderhalses (2) des Zylinderkörpers (1) von einer Zylinderkörperkappe (32) gedeckt ist.

15. Einziehbare selbstzerstörende Sicherheitsspritze mit austauschbarer Nadel nach Anspruch 1, **dadurch gekennzeichnet, dass**: eine ringförmige Eingriffsdichtungsausnehmung (33), die zum ringförmigen Eingriffsteil (10) passt, an einer Innenwand des Zylinderhalses (2) des Zylinderkörpers (1) angeordnet ist, die Tiefe der ringförmigen Eingriffsdichtungsausnehmung (33) im Bereich von 0,01 mm bis 4 mm liegt und die Höhe der ringförmigen Eingriffsdichtungsausnehmung (33) im Bereich von 0,01 mm bis 6 mm liegt, und ein Ausnehmungsboden der ringförmigen Eingriffsdichtungsausnehmung (33) eine gekrümmte Fläche ist, und drehungsverhindernde Rippen (34), die zu den drehungsverhindernden Rippen (13) passen, an der Innenwand des Zylinderhalses (2) unter der ringförmigen Eingriffsdichtungsausnehmung (33) geformt sind.

16. Einziehbare selbstzerstörende Sicherheitsspritze mit austauschbarer Nadel nach Anspruch 1, **dadurch gekennzeichnet, dass**: zumindest eine ringförmige Anschlagnut der Nadelbasis (35) an der Innenwand des Hohlraums des Nadelbasiskörpers (3) geformt ist, das innen eingehülste zurückziehbare Verriegelungselement (4) im Hohlraum des Nadelbasiskörpers (3) angeordnet ist, und zumindest ein Anschlagflanschring (36), der zur ringförmigen Anschlagnut in der Nadelbasis (35) passt, an einer Außenwand des innen eingehülsten zurückziehbaren Verriegelungselements (4) geformt ist.

17. Einziehbare selbstzerstörende Sicherheitsspritze mit austauschbarer Nadel nach Anspruch 1, **dadurch gekennzeichnet, dass**: das innen eingehülste zurückziehbare Verriegelungselement (4) einen leeren Innenhohlraum aufweist, ein ringförmiger Eingriffs- und Verriegelungsabschnitt (38) an der Innenwand des Hohlraums nahe bei einer Öffnung eines unteren Endes des innen eingehülsten zurückziehbaren Verriegelungselementes (4) geformt ist.

18. Einziehbare selbstzerstörende Sicherheitsspritze mit austauschbarer Nadel nach Anspruch 17, **dadurch gekennzeichnet, dass**: eine ringförmige Ausnehmung (37) an einem oberen Teil des ringförmigen Eingriffs- und Verriegelungsabschnitts (38) geformt ist, eine Dicke einer inneren Seitenwand (40) der ringförmigen Ausnehmung (37) im Bereich von 0,01 mm bis 3 mm liegt, ein Winkel im Bereich von 2 bis 90 Grad zwischen der inneren Seitenwand der ringförmigen Ausnehmung (37) und der äußeren Seitenwand (41) der ringförmigen Ausnehmung (37) geformt ist, und ein Innendurchmesser eines oberen Endes der inneren Seitenwand der ringförmigen Ausnehmung (37) im Bereich von 1 mm bis 80 mm liegt.

19. Einziehbare selbstzerstörende Sicherheitsspritze mit austauschbarer Nadel nach Anspruch 18, **dadurch gekennzeichnet, dass**: die Breite einer Öffnung der ringförmigen Ausnehmung (37) größer als die Breite eines Bodens derselben ist.

## Revendications

1. Seringue de sécurité à autodestruction à aiguille remplaçable, comprenant un corps de cylindre (1), un corps de base (3) d'aiguille disposé à l'intérieur d'un col de cylindre (2) au niveau d'une extrémité du corps de cylindre (1), un piston (5) situé dans le corps de cylindre (1), et un joint (6) en caoutchouc situé à proximité d'une extrémité du piston (5), **caractérisée en ce que** : un mécanisme d'engagement à amortissement est formé entre le col de cylindre (2) et le corps de base (3) d'aiguille, dans laquelle le mécanisme d'engagement à amortissement élastique est placé sur le corps de base (3) d'aiguille, comportant une cavité dans laquelle est placé un élément de verrouillage-rétraction de type manchon intérieur (4), ou une partie extérieure du corps de base (3) est appliquée sur un élément de verrouillage-rétraction à manchon ; un ensemble aiguille (7) remplaçable est formé sur une partie supérieure du corps de base (3) d'aiguille et un bouchon d'étanchéité (8) élastique est formé sur une partie inférieure du corps de base (3) d'aiguille, dans lequel l'ensemble aiguille (7) remplaçable et le bouchon d'étanchéité (8) élastique sont intégrés l'un à l'autre pour former une seule structure ; un espace annulaire d'amortissement (9) est défini par une surface du bouchon d'étanchéité (8) élastique ; une pièce d'engagement à anneau (10) est formée sur une paroi extérieure du bouchon d'étanchéité (8) élastique à l'extérieur de l'espace annulaire d'amortissement (9) ; des nervures anti-rotation (13) sont formées sur la paroi extérieure du bouchon d'étanchéité (8) élastique ; un ensemble filetage extérieur (11) est formé sur l'ensemble aiguille (7) remplaçable et un élément de verrouillage - rétraction élastique asymétrique est placé au niveau d'une extrémité du piston (5) ;
dans laquelle l'élément de verrouillage-rétraction élastique asymétrique est un crochet (12) en plastique, et le crochet est formé de façon intégrée avec le piston (5) dans une seule opération de moulage ; et
dans laquelle une partie de base (14) est formée au niveau d'une extrémité inférieure du crochet (12) ; un premier bras (15) dévié vers un côté est formé sur une partie supérieure de la partie de base (14) ; un angle courbe concave (16) ou un coin est défini par une paroi latérale extérieure d'une jonction entre le premier bras (15) et la partie de base (14), et l'angle courbe concave (16) est de 45 à 179 degrés ; une extrémité inférieure du premier bras (15) est reliée à une extrémité supérieure de la partie de base (14), une extrémité supérieure du premier bras (15) est reliée à une extrémité d'un deuxième bras (17) et une autre extrémité du deuxième bras (17) est suspendue ; une surface courbe (18) est formée sur le côté extérieur d'une jonction entre le premier bras (15) et le deuxième bras (17), un angle interne (39) de 2 a 90 degrés est défini par un côté intérieur de la jonction entre le premier bras (15) et le deuxième bras (17) ; une largeur de l'ouverture formée entre l'extrémité suspendue du deuxième bras (17) et le premier bras (15) est de 0,5 mm à 40 mm ; la longueur du premier bras (15) est de 0,5 mm à 50 mm, et la longueur du deuxième bras (17) est de 0,5 mm à 60 mm.

2. Seringue de sécurité à autodestruction à aiguille remplaçable selon la revendication 1, **caractérisée en ce que** : le mécanisme d'engagement à amortissement est placé sur le corps de base (3) d'aiguille, comportant une cavité dans laquelle est placé un élément de verrouillage-rétraction de type manchon intérieur (4), ou une partie extérieure du corps de base (3) est appliquée sur un élément de verrouillage-rétraction à manchon ; un ensemble aiguille (7) remplaçable est formé sur une partie supérieure du corps de base (3) d'aiguille et un bouchon d'étanchéité (8) élastique est formé sur une partie inférieure du corps de base (3) d'aiguille, dans lequel l'ensemble aiguille (7) remplaçable et le bouchon d'étanchéité (8) élastique sont des parties indépendantes pour former une structure d'ensemble fendue; un espace annulaire d'amortissement (9) est défini par une surface du bouchon d'étanchéité (8) élastique ; une pièce d'engagement (10) annulaire est formée sur une paroi extérieure du bouchon d'étanchéité (8) élastique à l'extérieur de l'espace annulaire d'amortissement (9) ; des nervures anti-rotation (13) sont formées sur la paroi extérieure du bouchon d'étanchéité (8) élastique ; un ensemble filetage extérieur (11) est formé sur l'ensemble aiguille (7) remplaçable.

3. Seringue de sécurité à autodestruction à aiguille remplaçable selon la revendication 1, **caractérisée en ce que** : un axe central de l'élément de verrouillage-rétraction élastique asymétrique se trouve sur une ligne centrale de symétrie du piston, une partie de verrouillage est respectivement formée sur les deux côtés de l'axe central et les parties de verrouillage sur les deux côtés sont asymétriques l'une par rapport à l'autre, l'une des parties de verrouillage sur un côté de l'axe central étant située à un niveau supérieur ou inférieur que l'autre partie de verrouillage sur l'autre côté, et la largeur de l'une des parties de verrouillage sur un côté de l'axe central est supérieure ou inférieure que la largeur de l'autre partie de verrouillage sur l'autre côté ; un mécanisme élastique est formé sur la partie de verrouillage sur un côté ou sur les parties de verrouillage sur les deux côtés, et une distance entre un bord extérieur de la partie de verrouillage sur un côté et la ligne centrale de symétrie du piston est comprise entre 1 mm et 20 mm.

4. Seringue de sécurité à autodestruction à aiguille remplaçable selon la revendication 1, **caractérisée en ce que** : quatre nervures de renforcement (21) disposées en croix de manière à former des angles égaux sont formées sur le piston (5), avec une pièce de pré-bouchon (22) disposée sur un bord extérieur des nervures de renforcement (21).

5. Seringue de sécurité à autodestruction à aiguille remplaçable selon la revendication 4, **caractérisée en ce que** : la pièce de pré-bouchon (22) est reliée aux nervures de renforcement (21) au moyen d'au moins deux parties de liaison (23).

6. Seringue de sécurité à autodestruction à aiguille remplaçable selon la revendication 1, **caractérisée en ce que** : une pointe (24) est formée sur l'extrémité suspendue du deuxième bras (17), et l'extrémité suspendue du deuxième bras (17) fait saillie du bord extérieur de la partie de base (14).

7. Seringue de sécurité à autodestruction à aiguille remplaçable selon la revendication 1, **caractérisée en ce que** : une embase (24) d'aiguille remplaçable est reliée à l'ensemble filetage extérieur (11) sur l'ensemble aiguille remplaçable (7), un tube d'aiguille (25) est placé sur un centre d'une partie supérieure de l'embase (24) d'aiguille remplaçable; une cavité (30) en forme de colonne reliée avec le tube d'aiguille (25) est formée dans l'embase (24) d'aiguille remplaçable, un ensemble filetage intérieur (31) emboité avec l'ensemble filetage extérieur (11) est formé sur une paroi interne de la cavité de l'embase (24) d'aiguille remplaçable, une partie supérieure de la cavité (30) en forme de colonne est un tronc de cône, et la conicité du tronc de cône est 6:100.

8. Seringue de sécurité à autodestruction à aiguille remplaçable selon la revendication 1, **caractérisée en ce que** : les nervures anti-rotation (13) sont distribuées uniformément en forme annulaire sur la paroi externe du bouchon d'étanchéité élastique (8) et les extrémités des nervures anti-rotation (13) sont des demi-cercles ou des cônes.

9. Seringue de sécurité à autodestruction à aiguille remplaçable selon la revendication 1, **caractérisée en ce que** : une partie supérieure de l'ensemble aiguille remplaçable (7) est un tronc de cône, la conicité du tronc de cône est 6:100 et l'ensemble filetage extérieur (11) est formé sur une paroi externe de la partie inférieure de l'ensemble aiguille remplaçable (7).

10. Seringue de sécurité à autodestruction à aiguille remplaçable selon la revendication 1, **caractérisée en ce que** : un gradin annulaire (26) ou une pente annulaire est formée sur un raccordement entre l'ensemble aiguille remplaçable (7) et le bouchon d'étanchéité élastique (8).

11. Seringue de sécurité à autodestruction à aiguille remplaçable selon la revendication 1, **caractérisée en ce que** : l'espace d'amortissement annulaire (9) est une structure à rainure annulaire, une hauteur de la rainure, de son ouverture à son fond, est de 0,05 mm à 4 mm, une largeur de l'espace d'amortissement annulaire (9) est de 0,05 à 4 mm, la distance d'une paroi externe de rainure de l'espace d'amortissement annulaire (9) à un sommet d'une surface courbe de la pièce d'engagement à anneau (10) est de 0,1 mm à 6 mm, la distance de l'ouverture de la paroi externe de rainure de l'espace d'amortissement annulaire (9) à la paroi externe de la pièce d'engagement à anneau (10) est de 0,1 mm à 6 mm, la distance du fond de la paroi externe de rainure de l'espace d'amortissement annulaire (9) à la paroi externe de la pièce d'engagement à anneau (10) est de 0,1 mm à 6 mm; une surface de la pièce d'engagement à anneau (10) est une surface courbe, et la distance du sommet de la surface courbe de la pièce d'engagement à anneau (10) à un bord supérieur de la surface courbe est inférieure à la distance du sommet de la surface courbe de la pièce d'engagement à anneau (10) à un bord inférieur de la surface courbe, et un évidement annulaire (27) est formé sur la paroi externe du bouchon d'étanchéité élastique (8) au-dessous de la pièce d'engagement à anneau (10).

12. Seringue de sécurité à autodestruction à aiguille remplaçable selon la revendication 11, **caractérisée en ce que** : l'évidement annulaire (27) est sur le même plan horizontal qu'un fond de rainure de l'espace d'amortissement annulaire (9), ou à un niveau supérieur que le fond de rainure de l'espace amortissement annulaire (9).

13. Seringue de sécurité à autodestruction à aiguille remplaçable selon la revendication 10, **caractérisée en ce que** : une rainure d'ensemble joint torique (28) est formée sur une paroi externe du bouchon d'étanchéité élastique (8) au-dessous du gradin annulaire (26), et un joint d'étanchéité torique (29) est assemblé dans la rainure d'ensemble joint torique (28).

14. Seringue de sécurité à autodestruction à aiguille remplaçable selon la revendication 1, **caractérisée en ce que** : le col de cylindre (2) du corps de cylindre (1) est couvert à son extérieur par un capuchon de corps de cylindre (32).

15. Seringue de sécurité à autodestruction à aiguille remplaçable selon la revendication 1, **caractérisée en ce que** : un évidement d'étanchéité d'engagement annulaire (33) correspondant à la pièce d'engagement à anneau (10) est disposé sur une paroi interne du col de cylindre (2) du corps de cylindre (1), la profondeur de l'évidement d'étanchéité d'engagement annulaire (33) est de 0,01 mm à 4 mm, et la hauteur de l'évidement d'étanchéité d'engagement annulaire (33) est de 0,01 mm à 6 mm, et un fond d'évidement de l'évidement d'étanchéité d'engagement annulaire (33) est une surface courbe, et des nervures anti-rotation (34) correspondant aux nervures anti-rotation (13) sont formées sur la paroi interne du col de cylindre (2) au-dessous de l'évidement d'étanchéité d'engagement annulaire (33).

16. Seringue de sécurité à autodestruction à aiguille remplaçable selon la revendication 1, **caractérisée en ce que** : au moins une rainure d'arrêt annulaire de l'embase d'aiguille (35) est formée sur la paroi interne de la cavité du corps d'embase d'aiguille (3), l'élément de verrouillage-rétraction de type manchon intérieur (4) est disposé dans la cavité du cors d'embase d'aiguille (3) est au moins une bride annulaire d'arrêt (36) correspondant à la rainure d'arrêt annulaire à l'intérieur de l'embase d'aiguille (35) est formée sur une paroi externe de l'élément de verrouillage-rétraction de type manchon intérieur (4).

17. Seringue de sécurité à autodestruction à aiguille remplaçable selon la revendication 1, **caractérisée en ce que** : l'élément de verrouillage-rétraction de type manchon intérieur (4) comporte une cavité intérieure, une partie de verrouillage et d'engagement à anneau (38) est formée sur la paroi interne de la cavité près d'une ouverture de l'extrémité inférieure de l'élément de verrouillage-rétraction de type manchon intérieur (4).

18. Seringue de sécurité à autodestruction à aiguille remplaçable selon la revendication 17, **caractérisée en ce que** : un évidement annulaire (37) est formé sur une partie supérieure de la partie de verrouillage et d'engagement à anneau (38), une épaisseur d'une paroi latérale interne (40) de l'évidement annulaire (37) est de 0,01 mm à 3 mm, un angle de 2 à 90 degrés est formé entre la paroi latérale interne de l'évidement annulaire (37) et une paroi latérale externe (41) de l'évidement annulaire (37) et un diamètre intérieur d'une extrémité supérieure de la paroi latérale interne de l'évidement annulaire (37) est compris entre 1 mm et 80 mm.

19. Seringue de sécurité à autodestruction à aiguille remplaçable selon la revendication 18, **caractérisée en ce que** : la largeur d'une ouverture de l'évidement annulaire (37) est supérieure à la largeur d'un fond de celui-ci.
